# FASCICULE DE BREVET EUROPEEN

(11) **EP 4 072 891 B1**
(45) Date de publication et mention de la délivrance du brevet: **17.07.2024**
(21) Numéro de dépôt: 20821333.0
(22) Date de dépôt: 18.11.2020
(51) Int. Cl.: B60K 35/00

(54) **DISPOSITIF DESTINÉ À LUTTER CONTRE LA CINÉTOSE INTÉGRÉ DANS UN VÉHICULE AUTOMOBILE**
IN EIN KRAFTFAHRZEUG INTEGRIERTE VORRICHTUNG GEGEN REISEKRANKHEIT
DEVICE FOR CONTROLLING MOTION SICKNESS, WHICH IS INTEGRATED INTO A MOTOR VEHICLE

(30) Priorité: 13.12.2019 FR 1914356
(43) Date de publication de la demande: 19.10.2022
(73) Titulaire: NOVARES France, 78140 Vélizy-Villacoublay (FR)
(72) Inventeur: CAZES, Christophe, 78000 VERSAILLES (FR); BOLLIER, François, 06300 NICE (FR)
(74) Mandataire: Germain Maureau
(86) Numéro de dépôt international: PCT/FR2020/052108
(87) Numéro de publication internationale: WO 2021/116550

(56) Documents cités:
- DE-A1- 102014 221 337
- DE-A1- 102017 207 788
- "Accelerometer", 10 November 2019 (2019-11-10), XP002800050, Retrieved from the Internet <URL:https://en.wikipedia.org/w/index.php?title=Accelerometer&oldid=925500489> [retrieved on 20200817]
- "Reisekrankheit", 28 July 2019 (2019-07-28), XP002800051, Retrieved from the Internet <URL:https://de.wikipedia.org/w/index.php?title=Reisekrankheit&oldid=190821601> [retrieved on 20200817]

## Description

L'invention concerne un dispositif destiné à lutter contre la cinétose intégré dans un véhicule automobile et un véhicule automobile équipé dudit dispositif.

Un problème couramment rencontré par les personnes voyageant dans un véhicule, un avion ou un bateau est le mal des transports, également appelé cinétose. Le mal des transports est dû à un décalage entre les sensations ressenties dans le système vestibulaire de l'oreille interne et celles éprouvées par d'autres sens, telles que les perceptions visuelles d'une personne. Les récepteurs d'équilibre dans l'oreille interne sont sensibles à la gravité (par exemple, aux changements d'orientation), à la vitesse et aux changements de vitesse (accélérations) se produisant lors du déplacement du véhicule. Lorsque les sensations ressenties par l'oreille interne ne correspondent pas aux signaux visuels perçus par la personne, il en résulte souvent un mal des transports pour la personne, se traduisant notamment sous la forme de nausées et maux de tête.

Par exemple, un passager voyageant sur une route sinueuse dans une automobile subit des accélérations linéaires et angulaires à chaque fois que le véhicule se déplace dans une courbe. La réponse du système de détection vestibulaire à l'accélération provoquée par le mouvement du véhicule ne correspondra pas à la perception visuelle à moins que la personne ne regarde continuellement la route, de sorte que la perception de son oreille interne corresponde à la trajectoire du véhicule perçue visuellement dans les courbes. C'est pour cette raison que le conducteur d'un véhicule ne souffre généralement pas du mal des transports, alors que les passagers du véhicule peuvent en souffrir. En effet, le conducteur surveille en permanence la route et perçoit visuellement le mouvement du véhicule de sorte que les perceptions visuelles correspondent aux sens de l'oreille interne. A l'inverse, les passagers d'un véhicule qui lisent ou ne regardent que l'intérieur du véhicule ou qui exercent d'autres activités qui les empêchent de surveiller la route auront une perception visuelle qui ne correspond pas à celle de leur oreille interne.

Pour éviter le mal des transports, une solution consiste donc pour le passager à surveiller la route comme s'il conduisait le véhicule, de sorte que les informations visuelles qu'il reçoit correspondent davantage aux sensations de son système vestibulaire. Cependant, lorsqu'il est assis à l'arrière du véhicule, il voit généralement la route de manière partielle. Le mouvement réel du passager perçu par l'appareil auditif interne de l'oreille vestibulaire ne peut donc pas être facilement associé à la perception visuelle de ce mouvement. Le mal des transports ne peut donc pas être évité dans ce cas.

Une autre solution au mal des transports, décrite dans le document US 2019/0083739, consiste à utiliser des repères lumineux dans les montants intérieurs d'un habitacle de véhicule, lesdits repères lumineux formant des colonnes lumineuses de chaque côté de l'habitacle. La hauteur des colonnes lumineuses est contrôlée par des moyens de commande électroniques de manière à créer un horizon artificiel correspondant à la perception de l'oreille interne d'un passager assis dans le véhicule. Cette solution présente toutefois l'inconvénient d'afficher les repères lumineux quel que soit le déplacement du véhicule. Ainsi, dans cette solution connue, un horizon artificiel est constamment affiché dans l'habitacle. Or, il est scientifiquement prouvé que la cinétose ne se produit que pour une gamme de fréquence d'accélération bien spécifique. En particulier, les premiers symptômes annonciateurs d'une cinétose surviennent généralement pour des fréquences d'accélération inférieures à 25 Hz. Les premiers signes d'inconfort chez les passagers ne se manifestent véritablement que dans les gammes de fréquences d'accélération comprises entre 4 et 8 Hz. Toutefois, La cinétose ne devient véritablement gênante pour les passagers que dans le cas où les accélérations dans le sens avant-arrière ou gauche-droite du véhicule ont une fréquence inférieure à 0.5 Hz ou dans le cas où les accélérations dans le sens vertical ont une fréquence inférieure à 2 Hz. De ce fait, la solution décrite dans le document US 2019/0083739 présente l'inconvénient de procéder à un affichage de repères lumineux à l'intérieur du véhicule même lorsque le risque de cinétose est nul ou quasi-nul. Cet affichage intempestif peut donc gêner les passagers s'ils ne souffrent pas encore de cinétose. Par ailleurs, l'utilisation en continu de sources lumineuses pour l'affichage des repères lumineux génère une surconsommation d'énergie électrique laquelle, notamment dans le cas d'un véhicule électrique par exemple, peut avoir un impact non négligeable sur l'autonomie du véhicule.

D'autres solutions au mal des transports ont été décrites dans les documents DE 10 2007 207 788 A, DE 10 2014 221 337 A et Wikipédia « Accelerometer ».

L'invention vise donc à proposer un dispositif destiné à lutter contre la cinétose intégré dans un véhicule automobile et ne présentant pas les inconvénients de l'art antérieur susmentionné.

A cet effet, l'invention concerne un dispositif anti-cinétose équipant un véhicule automobile, le dispositif anti-cinétose comprenant :
- un accéléromètre triaxial configuré pour détecter les accélérations du véhicule selon 3 axes et émettre un signal d'accélération correspondant ;
- des moyens d'affichage de repères lumineux aptes à former au moins une première ligne d'horizon artificiel au niveau d'une première surface intérieure du véhicule automobile et au moins une deuxième ligne d'horizon artificiel au niveau d'une deuxième surface intérieure du véhicule automobile, les dites première et deuxième lignes d'horizon artificiel étant perpendiculaires ou sensiblement perpendiculaires l'une à l'autre ;
- une unité de contrôle apte à recevoir les signaux d'accélération émis par l'accéléromètre et à piloter les moyens d'affichage de telle sorte que les première et deuxième lignes d'horizon artificiel soient alignées dans un plan horizontal, perpendiculaire ou sensiblement perpendiculaire au vecteur gravitation, quelles que soient les accélérations du véhicule, l'unité de contrôle étant par ailleurs configurée pour traiter lesdits signaux d'accélération, préalablement à la commande des moyens d'affichage, de manière à déterminer en temps réel une fréquence d'accélération selon chacun des axes de l'accéléromètre et à piloter lesdits moyens d'affichage uniquement lorsque ladite fréquence d'accélération est inférieure à une fréquence seuil en-dessous de laquelle la cinétose est susceptible de se produire.

Ainsi configuré, le dispositif anti-cinétose de l'invention permet de supprimer le mal des transports en affichant deux lignes d'horizon artificiel, respectivement l'une en face d'une personne assise dans le véhicule et l'autre à côté de ladite personne, lesdites lignes étant alignées dans un plan horizontal. Le dispositif permet en outre de n'afficher les repères lumineux que pour les gammes de fréquence d'accélération où le risque de cinétose est élevé.

Le dispositif de l'invention pourra également comprendre une ou plusieurs des caractéristiques suivantes :
- la fréquence seuil est égale à 25 Hz.
- la fréquence seuil est comprise entre 4 et 8 Hz.
- la fréquence seuil est égale à 0.5 Hz pour les accélérations selon un axe longitudinal, correspondant à la direction avant-arrière du véhicule, et selon un axe latéral, correspondant à la direction droite-gauche du véhicule, et est égale à 2 Hz pour les accélérations selon un axe vertical, correspondant à la direction haut-bas du véhicule.
- les moyens d'affichage sont aptes à émettre au moins deux faisceaux lumineux de forme rectiligne, respectivement un premier faisceau lumineux projeté sur la première surface intérieure et formant la première ligne d'horizon artificiel, et un deuxième faisceau lumineux projeté sur la deuxième surface intérieure et formant la deuxième ligne d'horizon artificiel.
- les moyens d'affichage comprennent au moins une source lumineuse émettant un faisceau lumineux principal, et des moyens de séparation et de déviation dudit faisceau lumineux principal en deux faisceaux lumineux secondaires.
- les moyens de séparation et de déviation comprennent un prisme destiné à séparer le faisceau lumineux en deux faisceaux lumineux secondaires et une combinaison de miroirs et/ou de lentilles destinée à modifier la trajectoire desdits faisceaux lumineux secondaires.
- les moyens d'affichage comprennent au moins une paire de sources lumineuses, respectivement une première source lumineuse émettant le premier faisceau lumineux et une deuxième source lumineuse émettant le deuxième faisceau lumineux.
- la source lumineuse, respectivement la paire de sources lumineuses, est un laser, respectivement une paire de lasers.
- les moyens d'affichage comprennent au moins trois colonnes lumineuses orientées verticalement, respectivement une première colonne lumineuse disposée dans l'alignement avec la première surface intérieure et dans l'alignement avec la deuxième surface intérieure, une deuxième colonne lumineuse disposée dans l'alignement avec la deuxième surface intérieure et à gauche ou à droite de la première colonne lumineuse, et une troisième colonne lumineuse disposée dans l'alignement avec la première surface intérieure et plus proche de l'arrière du véhicule que la première colonne lumineuse, chacune des colonnes lumineuses étant formée d'une pluralité de points lumineux alignés dans le sens vertical, chacun des points lumineux pouvant émettre une lumière dans un état activé et ne pas émettre de lumière dans un état désactivé, et en ce que la première ligne d'horizon artificiel est formée par la droite virtuelle passant par les points lumineux activés les plus hauts, ou les plus bas, sur les première et troisième colonnes lumineuses respectivement et la deuxième ligne d'horizon artificiel est formée par la droite virtuelle passant par les points lumineux activés les plus hauts, ou les plus bas, sur les première et deuxième colonnes lumineuses respectivement.
- chacune des colonnes lumineuses comprend un réseau linéaire de diodes électroluminescentes alignées verticalement, chacune des diodes électroluminescentes formant un point lumineux.

L'invention concerne également un véhicule automobile équipé du dispositif anti-cinétose tel que défini précédemment.

Dans une configuration particulière de l'invention, le véhicule comprend au moins un montant central, ledit montant central supportant les moyens d'affichage, lesdits moyens d'affichage étant configurés pour projeter le premier faisceau lumineux sur une vitre latérale arrière ou un panneau de porte arrière du véhicule et pour projeter le deuxième faisceau lumineux sur une surface intérieure de l'habitacle disposée de manière sensiblement perpendiculaire à ladite vitre ou audit panneau de porte, par exemple le dossier d'un des sièges avant du véhicule.

L'invention concerne également un procédé d'affichage de repères lumineux destinés à lutter contre la cinétose, comprenant les étapes suivantes :
- détection au moyen d'un accéléromètre triaxial des accélérations d'un véhicule selon 3 axes et envoi de signaux correspondants à une unité de contrôle ;
- détermination en temps réel d'une fréquence d'accélération selon chacun des axes de l'accéléromètre et comparaison entre la fréquence d'accélération déterminée et une fréquence seuil en-dessous de laquelle la cinétose est susceptible de se produire ;
- dans le cas où la fréquence d'accélération déterminée est inférieure à la fréquence seuil, pilotage par l'unité de contrôle de moyens d'affichage de repères lumineux aptes à former au moins une première ligne d'horizon artificiel au niveau d'une première surface intérieure du véhicule automobile et au moins une deuxième ligne d'horizon artificiel au niveau d'une deuxième surface intérieure du véhicule automobile, les dites première et deuxième lignes d'horizon artificiel étant perpendiculaires ou sensiblement perpendiculaires l'une à l'autre, de telle sorte que les première et deuxième lignes d'horizon artificiel soient alignées dans un plan horizontal, perpendiculaire au vecteur gravitation, quelles que soient les accélérations du véhicule.

D'autres caractéristiques et avantages ressortiront clairement de la description ci-après de deux modes particuliers de réalisation de l'invention, donnés à titre d'exemples non limitatifs, en référence aux dessins annexés dans lesquels :
[Fig. 1] est une vue en perspective de l'habitacle d'un véhicule selon un premier mode de réalisation de l'invention ;
[Fig. 2] est une vue en perspective de l'habitacle d'un véhicule selon un deuxième mode de réalisation de l'invention ;
[Fig. 3] est une vue arrière d'un véhicule selon l'invention, le véhicule étant soumis à une accélération selon l'axe Y;
[Fig. 4] est une vue latérale d'un véhicule selon l'invention, le véhicule étant soumis à une accélération selon l'axe X;
[Fig. 5] est une vue arrière d'un véhicule selon l'invention, le véhicule étant soumis à une accélération selon l'axe Y.

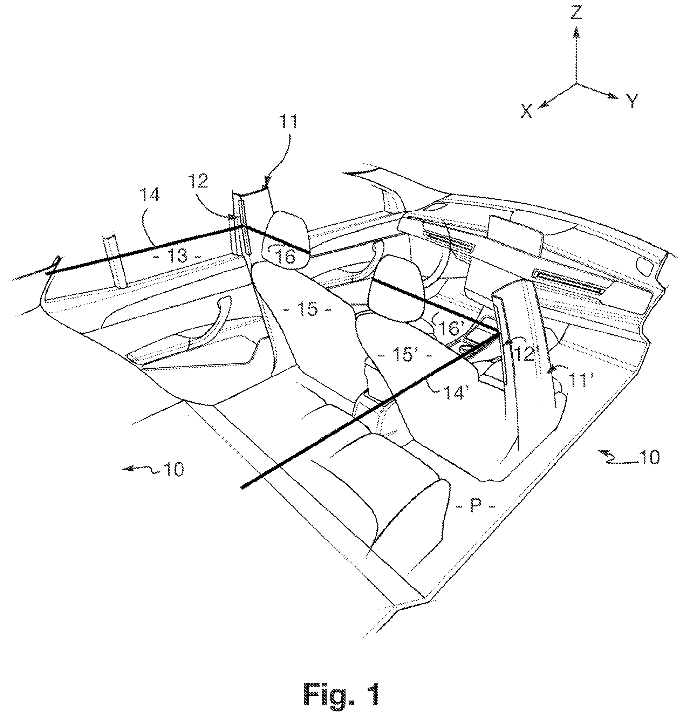

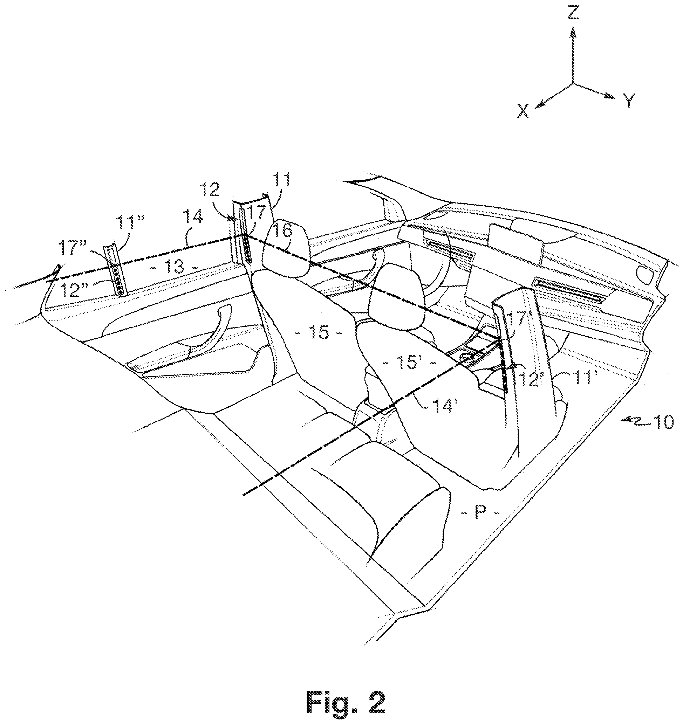

Dans la suite de cette description, et en référence au repère cartésien XYZ représenté sur les figures 1 et 2, il sera utilisé les termes de « direction longitudinale » pour une direction selon l'axe X, de « direction transversale » pour une direction selon l'axe Y et de « direction verticale » pour une direction selon l'axe Z. Par ailleurs, par convention, le terme « avant » sera utilisé pour indiquer une orientation dirigée vers l'avant du véhicule et le terme « arrière » sera utilisé pour indiquer une orientation dirigée vers l'arrière du véhicule.

En référence à la fig. 1, il est représenté un habitacle d'un véhicule 10 selon un premier mode de réalisation de l'invention. Dans ce mode de réalisation, une première colonne d'éclairage 12 orientée verticalement est supportée par un montant central 11 du véhicule et est disposée du côté gauche par rapport au champ de vision d'un passager assis à l'arrière du véhicule, et une deuxième colonne d'éclairage 12' orientée verticalement est supportée par un autre montant central 11' du véhicule et est disposée du côté droit par rapport au champ de vision d'un passager assis à l'arrière du véhicule. Chaque colonne d'éclairage 12, 12' comprend au moins une source lumineuse configurée pour projeter un premier faisceau lumineux rectiligne sur une vitre latérale arrière ou un panneau de porte arrière 13 du véhicule et pour projeter un deuxième faisceau lumineux rectiligne sur le dossier d'un des sièges avant 15 ou 15' du véhicule. Le premier faisceau lumineux émis par la première colonne d'éclairage 12 se projette sur la vitre latérale arrière ou le panneau de porte arrière 13 directement adjacent à la première colonne d'éclairage 12 selon une première ligne 14, référencée par la suite en tant que première ligne d'horizon artificiel, et le deuxième faisceau lumineux émis par la première colonne d'éclairage 12 se projette sur le dossier du siège avant 15 directement adjacent à la première colonne d'éclairage 12 selon une deuxième ligne 16, référencée par la suite en tant que deuxième ligne d'horizon artificiel. De manière similaire, le premier faisceau lumineux émis par la deuxième colonne d'éclairage 12' se projette sur la vitre latérale arrière ou le panneau de porte arrière (non représenté) directement adjacent à la deuxième colonne d'éclairage 12' selon une première ligne 14', référencée par la suite en tant que première ligne d'horizon artificiel, et le deuxième faisceau lumineux émis par la deuxième colonne d'éclairage 12' se projette sur le dossier du siège avant 15' directement adjacent à la deuxième colonne d'éclairage 12' selon une deuxième ligne 16', référencée par la suite en tant que deuxième ligne d'horizon artificiel. Dans cette configuration, les première et deuxième lignes d'horizon artificiel sont perpendiculaires ou sensiblement perpendiculaires l'une à l'autre.

En pilotant de manière adaptée la ou les sources lumineuses, il est ainsi possible d'aligner les première et deuxième lignes d'horizon artificiel 14, 16 et 14', 16' dans un plan horizontal qui est toujours perpendiculaire au vecteur gravitation. Ainsi, une personne assise à côté de la vitre latérale arrière 13 et derrière le siège avant 15 et fixant lesdites lignes d'horizon artificiel 14, 16 aura les mêmes sensations visuelles que le conducteur regardant la route : il ne sera donc plus soumis au mal des transports. Pour aboutir à ce résultat, le véhicule 10 est avantageusement équipé d'un accéléromètre triaxial configuré pour détecter les accélérations du véhicule selon les 3 axes X, Y et Z et émettre des signaux d'accélération correspondants, et d'une unité de contrôle apte à recevoir les signaux d'accélération émis par l'accéléromètre et à piloter la ou les sources lumineuses de telle sorte que les première et deuxième lignes d'horizon artificiel 14, 16 et 14', 16' soient alignées dans un plan horizontal A, perpendiculaire au vecteur gravitation, quelles que soient les accélérations du véhicule, l'unité de contrôle étant par ailleurs configurée pour traiter lesdits signaux d'accélération, préalablement au contrôle des sources lumineuses, de manière à déterminer en temps réel une fréquence d'accélération selon chacun des axes de l'accéléromètre et à piloter lesdites sources lumineuses uniquement lorsque ladite fréquence d'accélération est inférieure à une fréquence seuil en-dessous de laquelle la cinétose est susceptible de se produire.

En référence à la fig. 2, il est représenté un habitacle d'un véhicule selon un deuxième mode de réalisation de l'invention. Dans ce mode de réalisation, le dispositif anti-cinétose comprend au moins trois colonnes lumineuses orientées verticalement, respectivement une première colonne lumineuse 12 qui est supportée par un montant central 11 du véhicule et qui est disposée du côté gauche par rapport au champ de vision d'un passager assis à l'arrière du véhicule, une deuxième colonne lumineuse 12' qui est supportée par un autre montant central 11' du véhicule et qui est disposée du côté droit par rapport au champ de vision d'un passager assis à l'arrière du véhicule, et une troisième colonne lumineuse 12" qui est supportée par un montant latéral 11" faisant partie d'une vitre latérale arrière ou d'un panneau de porte arrière 13 et du côté gauche par rapport au champ de vision d'un passager assis à l'arrière du véhicule. Chaque colonne lumineuse 12, 12', 12" est formée d'une pluralité de points lumineux alignés dans le sens vertical, chacun des points lumineux pouvant émettre une lumière dans un état activé et ne pas émettre de lumière dans un état désactivé. Ainsi, une première ligne d'horizon artificiel 14 est formée par la droite virtuelle passant par les points lumineux activés les plus hauts 17 et 17" sur les première et troisième colonnes lumineuses 12, 12" respectivement, et une deuxième ligne d'horizon artificiel 16 est formée par la droite virtuelle passant par les points lumineux activés les plus hauts 17 et 17' sur les première et deuxième colonnes lumineuses 12, 12' respectivement. Dans cette configuration, les première et deuxième lignes d'horizon artificiel 14, 16 sont perpendiculaires ou sensiblement perpendiculaires l'une à l'autre. Dans une variante avantageuse de l'invention, chacune des colonnes lumineuses 12, 12' et 12" pourra comprendre par exemple un réseau linéaire de diodes électroluminescentes alignées verticalement, chacune des diodes électroluminescentes formant l'un des points lumineux des colonnes lumineuses. Dans une autre configuration de l'invention, les points lumineux pourront être activé en partant du haut de chaque colonne lumineuse. Dans ce cas, la première ligne d'horizon artificiel 14 sera formée par la droite virtuelle passant par les points lumineux activés les plus bas sur les première et troisième colonnes lumineuses 12, 12" respectivement, et la deuxième ligne d'horizon artificiel 16 sera formée par la droite virtuelle passant par les points lumineux activés les plus bas sur les première et deuxième colonnes lumineuses 12, 12' respectivement. Par ailleurs, le véhicule 10 pourra avantageusement comprendre une quatrième colonne lumineuse (non représentée) qui sera supportée par un montant latéral faisant partie d'une vitre latérale arrière ou d'un panneau de porte arrière et du côté droit par rapport au champ de vision d'un passager assis à l'arrière du véhicule, ladite quatrième colonne lumineuse permettant de définir, en combinaison avec la deuxième colonne lumineuse 12', une troisième ligne d'horizon artificiel 14' sur le côté droit du véhicule.

En pilotant de manière adaptée les diodes électroluminescentes des première, deuxième et troisième colonnes lumineuses 12, 12' et 12", il est ainsi possible d'aligner les première et deuxième lignes d'horizon artificiel 14, 16 dans un plan horizontal qui est toujours perpendiculaire au vecteur gravitation. Ainsi, une personne assise à côté d'une vitre latérale arrière ou d'un panneau de porte latérale arrière 13 et derrière le siège avant 15 et fixant lesdites lignes d'horizon artificiel 14, 16 aura les mêmes sensations visuelles que le conducteur regardant la route : il ne sera donc plus soumis au mal des transports. L'unité de contrôle sera par ailleurs configurée pour traiter lesdits signaux d'accélération, préalablement au pilotage des diodes électroluminescentes, de manière à déterminer en temps réel une fréquence d'accélération selon chacun des axes de l'accéléromètre et à piloter lesdites diodes électroluminescentes uniquement lorsque ladite fréquence d'accélération est inférieure à une fréquence seuil en-dessous de laquelle la cinétose est susceptible de se produire

Les deux modes de réalisations décrits précédemment ne sont évidemment pas limitatifs pour l'invention. D'autres variantes de réalisation pourront être envisagées à ce niveau.

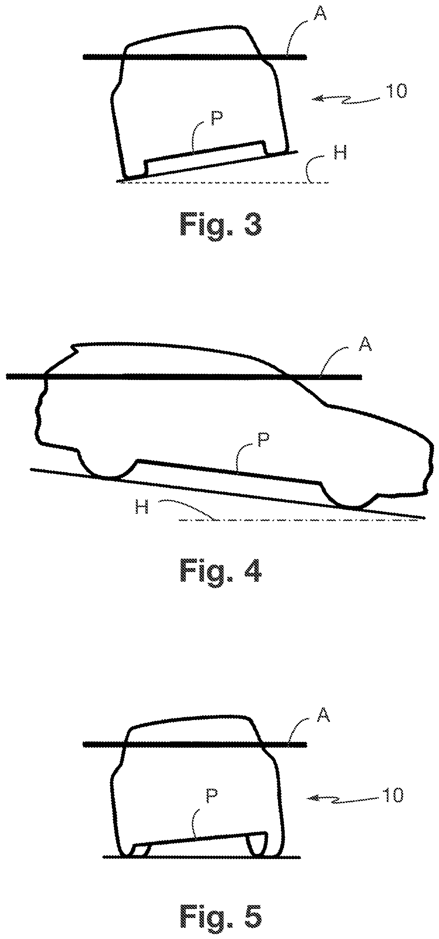

Les figs 3 à 5 illustrent plusieurs conditions de conduite possibles et le fonctionnement correspondant du dispositif anti-cinétose de l'invention.

Ainsi, dans le cas où le véhicule 10 circule sur une route inclinée sur la gauche par rapport à un plan horizontal fixe H, perpendiculaire au vecteur gravitation, comme représenté sur la fig. 3, il est soumis à une accélération selon l'axe Y. Cette accélération est détectée par l'accéléromètre triaxial, lequel envoie un signal d'accélération correspondant à l'unité de contrôle. En réponse à ce signal d'accélération, l'unité de contrôle détermine en temps réel une fréquence d'accélération selon chacun des axes de l'accéléromètre et compare cette fréquence d'accélération avec une fréquence seuil en-dessous de laquelle la cinétose est susceptible de se produire. Dans le cas présent, la fréquence d'accélération déterminée en temps réel selon l'axe Y étant inférieure à la fréquence seuil, l'unité de contrôle commande l'affichage des repères lumineux destinés à former les lignes d'horizon artificiel de telle sorte qu'elles soient alignées dans un plan horizontal A incliné par rapport au plancher P du véhicule au niveau de l'axe Y.

Dans le cas où le véhicule 10 circule sur une route inclinée vers le bas par rapport à un plan horizontal fixe H, perpendiculaire au vecteur gravitation, comme représenté sur la fig. 4, il est soumis à une accélération selon l'axe X. Cette accélération est détectée par l'accéléromètre triaxial, lequel envoie un signal d'accélération correspondant à l'unité de contrôle. En réponse à ce signal d'accélération, l'unité de contrôle détermine en temps réel une fréquence d'accélération selon chacun des axes de l'accéléromètre et compare cette fréquence d'accélération avec une fréquence seuil en-dessous de laquelle la cinétose est susceptible de se produire. Dans le cas présent, la fréquence d'accélération déterminée en temps réel selon l'axe X étant inférieure à la fréquence seuil, l'unité de contrôle commande l'affichage des repères lumineux destinés à former les lignes d'horizon artificiel de telle sorte qu'elles soient alignées dans un plan horizontal A incliné par rapport au plancher P du véhicule au niveau de l'axe X.

Dans le cas où le véhicule 10 circule sur une route plane et effectue un virage à gauche, comme représenté sur la fig. 5, il est soumis à une accélération selon l'axe Y. Cette accélération est détectée par l'accéléromètre triaxial, lequel envoie un signal d'accélération correspondant à l'unité de contrôle. En réponse à ce signal d'accélération, l'unité de contrôle détermine en temps réel une fréquence d'accélération selon chacun des axes de l'accéléromètre et compare cette fréquence d'accélération avec une fréquence seuil en-dessous de laquelle la cinétose est susceptible de se produire. Dans le cas présent, la fréquence d'accélération déterminée en temps réel selon l'axe Y étant inférieure à la fréquence seuil, l'unité de contrôle commande l'affichage des repères lumineux destinés à former les lignes d'horizon artificiel de telle sorte qu'elles soient alignées dans un plan horizontal A incliné par rapport au plancher P du véhicule au niveau de l'axe Y.

Par ailleurs, l'invention a également pour objet de protéger un procédé d'affichage de repères lumineux destinés à lutter contre la cinétose. Ce procédé pourra notamment comprendre les étapes suivantes :
- détection au moyen d'un accéléromètre triaxial des accélérations d'un véhicule selon 3 axes et envoi de signaux correspondants à une unité de contrôle ;
- détermination en temps réel d'une fréquence d'accélération selon chacun des axes de l'accéléromètre et comparaison entre la fréquence d'accélération déterminée et une fréquence seuil en-dessous de laquelle la cinétose est susceptible de se produire ;
- dans le cas où la fréquence d'accélération déterminée est inférieure à la fréquence seuil, pilotage par l'unité de contrôle de moyens d'affichage de repères lumineux aptes à former au moins une première ligne d'horizon artificiel au niveau d'une première surface intérieure du véhicule automobile et au moins une deuxième ligne d'horizon artificiel au niveau d'une deuxième surface intérieure du véhicule automobile, les dites première et deuxième lignes d'horizon artificiel étant perpendiculaires ou sensiblement perpendiculaires l'une à l'autre, de telle sorte que les première et deuxième lignes d'horizon artificiel soient alignées dans un plan horizontal, perpendiculaire au vecteur gravitation, quelles que soient les accélérations du véhicule.

## Revendications

1. Dispositif anti-cinétose équipant un véhicule automobile (10), **caractérisé en ce que** le dispositif anti-cinétose comprend :
- un accéléromètre triaxial configuré pour détecter les accélérations du véhicule (10) selon 3 axes et émettre un signal d'accélération correspondant ;
- des moyens d'affichage (12, 12', 12") de repères lumineux aptes à former au moins une première ligne d'horizon artificiel (14, 14') au niveau d'une première surface intérieure (13) du véhicule automobile et au moins une deuxième ligne d'horizon artificiel (16, 16') au niveau d'une deuxième surface intérieure (15, 15') du véhicule automobile, les dites première et deuxième lignes d'horizon artificiel (14, 16 ; 14', 16') étant perpendiculaires ou sensiblement perpendiculaires l'une à l'autre ;
- une unité de contrôle apte à recevoir les signaux d'accélération émis par l'accéléromètre et à piloter les moyens d'affichage (12, 12', 12") de telle sorte que les première et deuxième lignes d'horizon artificiel (14, 16 ; 14', 16') soient alignées dans un plan horizontal (A), perpendiculaire ou sensiblement perpendiculaire au vecteur gravitation, quelles que soient les accélérations du véhicule, l'unité de contrôle étant par ailleurs configurée pour traiter lesdits signaux d'accélération, préalablement à la commande des moyens d'affichage (12, 12', 12"), de manière à déterminer en temps réel une fréquence d'accélération selon chacun des axes de l'accéléromètre et à piloter lesdits moyens d'affichage (12, 12', 12") uniquement lorsque ladite fréquence d'accélération est inférieure à une fréquence seuil en-dessous de laquelle la cinétose est susceptible de se produire.

2. Dispositif selon la revendication 1, **caractérisé en ce que** la fréquence seuil est égale à 25 Hz.

3. Dispositif selon la revendication 1, **caractérisé en ce que** la fréquence seuil est comprise entre 4 et 8 Hz.

4. Dispositif selon la revendication 1, **caractérisé en ce que** la fréquence seuil est égale à 0.5 Hz pour les accélérations selon un axe longitudinal, correspondant à la direction avant-arrière du véhicule, et selon un axe latéral, correspondant à la direction droite-gauche du véhicule, et est égale à 2 Hz pour les accélérations selon un axe vertical, correspondant à la direction haut-bas du véhicule.

5. Dispositif selon l'une des revendications précédentes, **caractérisé en ce que** les moyens d'affichage (12, 12', 12") sont aptes à émettre au moins deux faisceaux lumineux de forme rectiligne, respectivement un premier faisceau lumineux projeté sur la première surface intérieure (13) et formant la première ligne d'horizon artificiel (14, 14'), et un deuxième faisceau lumineux projeté sur la deuxième surface intérieure (15, 15') et formant la deuxième ligne d'horizon artificiel (16, 16').

6. Dispositif selon la revendication 5, **caractérisé en ce que** les moyens d'affichage (12, 12', 12") comprennent au moins une source lumineuse émettant un faisceau lumineux principal, et des moyens de séparation et de déviation dudit faisceau lumineux principal en deux faisceaux lumineux secondaires.

7. Dispositif selon la revendication 6, **caractérisé en ce que** les moyens de séparation et de déviation comprennent un prisme destiné à séparer le faisceau lumineux en deux faisceaux lumineux secondaires et une combinaison de miroirs et/ou de lentilles destinée à modifier la trajectoire desdits faisceaux lumineux secondaires.

8. Dispositif selon la revendication 5, **caractérisé en ce que** les moyens d'affichage (12, 12', 12") comprennent au moins une paire de sources lumineuses, respectivement une première source lumineuse émettant le premier faisceau lumineux et une deuxième source lumineuse émettant le deuxième faisceau lumineux.

9. Dispositif selon l'une des revendications 6 à 8, **caractérisé en ce que** la source lumineuse, respectivement la paire de sources lumineuses, est un laser, respectivement une paire de lasers.

10. Dispositif selon l'une des revendications 1 à 4, **caractérisé en ce que** les moyens d'affichage comprennent au moins trois colonnes lumineuses (12, 12', 12") orientées verticalement, respectivement une première colonne lumineuse (12) disposée dans l'alignement avec la première surface intérieure (13) et dans l'alignement avec la deuxième surface intérieure (15, 15'), une deuxième colonne lumineuse (12') disposée dans l'alignement avec la deuxième surface intérieure (15, 15') et à gauche ou à droite de la première colonne lumineuse (12), et une troisième colonne lumineuse (12") disposée dans l'alignement avec la première surface intérieure (13) et plus proche de l'arrière du véhicule que la première colonne lumineuse (12), chacune des colonnes lumineuses (12, 12', 12") étant formée d'une pluralité de points lumineux alignés dans le sens vertical, chacun des points lumineux pouvant émettre une lumière dans un état activé et ne pas émettre de lumière dans un état désactivé, et **en ce que** la première ligne d'horizon artificiel (14) est formée par la droite virtuelle passant par les points lumineux activés (17, 17") les plus hauts, ou les plus bas, sur les première et troisième colonnes lumineuses (12, 12") respectivement et la deuxième ligne d'horizon artificiel (16) est formée par la droite virtuelle passant par les points lumineux activés (17, 17') les plus hauts, ou les plus bas, sur les première et deuxième colonnes lumineuses (12, 12') respectivement.

11. Dispositif selon la revendication 10, **caractérisé en ce que** chacune des colonnes lumineuses (12, 12', 12") comprend un réseau linéaire de diodes électroluminescentes alignées verticalement, chacune des diodes électroluminescentes formant un point lumineux.

12. Véhicule automobile (10) équipé d'un dispositif anti-cinétose selon l'une des revendications précédentes.

13. Véhicule automobile (10) équipé d'un dispositif anti-cinétose selon l'une des revendications 5 à 9, **caractérisé en ce qu'**il comprend au moins un montant central (11, 11'), ledit montant central supportant les moyens d'affichage (12, 12'), lesdits moyens d'affichage étant configurés pour projeter le premier faisceau lumineux sur une vitre latérale arrière ou un panneau (13) de porte arrière du véhicule et pour projeter le deuxième faisceau lumineux sur une surface intérieure (15, 15') de l'habitacle disposée de manière sensiblement perpendiculaire à ladite vitre ou audit panneau (13) de porte, par exemple le dossier d'un des sièges avant du véhicule.

14. Procédé d'affichage de repères lumineux destinés à lutter contre la cinétose, comprenant les étapes suivantes :
- détection au moyen d'un accéléromètre triaxial des accélérations d'un véhicule selon 3 axes et envoi de signaux correspondants à une unité de contrôle ;
- détermination en temps réel d'une fréquence d'accélération selon chacun des axes de l'accéléromètre et comparaison entre la fréquence d'accélération déterminée et une fréquence seuil en-dessous de laquelle la cinétose est susceptible de se produire ;
- dans le cas où la fréquence d'accélération déterminée est inférieure à la fréquence seuil, pilotage par l'unité de contrôle de moyens d'affichage (12, 12', 12") de repères lumineux aptes à former au moins une première ligne d'horizon artificiel (14, 14') au niveau d'une première surface intérieure (13) du véhicule automobile et au moins une deuxième ligne d'horizon artificiel (16, 16') au niveau d'une deuxième surface intérieure (15, 15') du véhicule automobile, les dites première et deuxième lignes d'horizon artificiel (14, 16 ; 14', 16') étant perpendiculaires ou sensiblement perpendiculaires l'une à l'autre, de telle sorte que les première et deuxième lignes d'horizon artificiel (14, 16 ; 14', 16') soient alignées dans un plan horizontal (A), perpendiculaire au vecteur gravitation, quelles que soient les accélérations du véhicule.

## Patentansprüche

1. Anti-Kinetose-Vorrichtung, mit der ein Kraftfahrzeug (10) ausgestattet ist, **dadurch gekennzeichnet, dass** die Anti-Kinetose-Vorrichtung Folgendes umfasst:
- einen dreiachsigen Beschleunigungsmesser, der dazu konfiguriert ist, die Beschleunigungen des Fahrzeugs (10) entlang 3 Achsen zu erfassen und ein entsprechendes Beschleunigungssignal zu emittieren;
- Anzeigemittel (12, 12', 12") für Lichtmarkierungen, die dazu ausgelegt sind, mindestens eine erste künstliche Horizontlinie (14, 14') auf der Höhe einer ersten Innenfläche (13) des Kraftfahrzeugs und mindestens eine zweite künstliche Horizontlinie (16, 16') auf der Höhe einer zweiten Innenfläche (15, 15') des Kraftfahrzeugs zu bilden, wobei die erste und die zweite künstliche Horizontlinie (14, 16; 14', 16') senkrecht oder im Wesentlichen senkrecht zueinander sind;
- eine Steuereinheit, die dazu ausgelegt ist, die vom Beschleunigungsmesser emittierten Beschleunigungssignale zu empfangen und die Anzeigemittel (12, 12', 12") so anzusteuern, dass die erste und die zweite künstliche Horizontlinie (14, 16; 14', 16') unabhängig von den Beschleunigungen des Fahrzeugs in einer horizontalen Ebene (A) senkrecht oder im Wesentlichen senkrecht zum Gravitationsvektor ausgerichtet sind, wobei die Steuereinheit des Weiteren dazu konfiguriert ist, die Beschleunigungssignale vor dem Bedienen der Anzeigemittel (12, 12', 12") zu verarbeiten, um eine Beschleunigungsfrequenz entlang jeder der Achsen des Beschleunigungsmessers in Echtzeit zu bestimmen und die Anzeigemittel (12, 12', 12") nur dann anzusteuern, wenn die Beschleunigungsfrequenz niedriger ist als eine Schwellenfrequenz, unterhalb derer die Kinetose wahrscheinlich auftritt.

2. Vorrichtung nach Anspruch 1, **dadurch gekennzeichnet, dass** die Schwellenfrequenz gleich 25 Hz ist.

3. Vorrichtung nach Anspruch 1, **dadurch gekennzeichnet, dass** die Schwellenfrequenz zwischen 4 und 8 Hz beträgt.

4. Vorrichtung nach Anspruch 1, **dadurch gekennzeichnet, dass** die Schwellenfrequenz gleich 0,5 Hz ist für die Beschleunigungen entlang einer Längsachse, die der Vorwärts-Rückwärts-Richtung des Fahrzeugs entspricht, und entlang einer Querachse, die der Links-Rechts-Richtung des Fahrzeugs entspricht, und gleich 2 Hz ist für Beschleunigungen entlang einer vertikalen Achse, die der Aufwärts-Abwärts-Richtung des Fahrzeugs entspricht.

5. Vorrichtung nach einem der vorhergehenden Ansprüche, **dadurch gekennzeichnet, dass** die Anzeigemittel (12, 12', 12") dazu ausgelegt sind, mindestens zwei Lichtstrahlen mit geradliniger Form, jeweils einen ersten Lichtstrahl, der auf die erste Innenfläche (13) projiziert wird und die erste künstliche Horizontlinie (14, 14') bildet, und einen zweiten Lichtstrahl, der auf die zweite Innenfläche (15, 15') projiziert wird und die zweite künstliche Horizontlinie (16, 16') bildet, zu emittieren.

6. Vorrichtung nach Anspruch 5, **dadurch gekennzeichnet, dass** die Anzeigemittel (12, 12', 12") mindestens eine Lichtquelle, die einen Hauptlichtstrahl ausgeben, und Mittel zum Trennen und Ablenken des Hauptlichtstrahls in zwei sekundäre Lichtstrahlen umfassen.

7. Vorrichtung nach Anspruch 6, **dadurch gekennzeichnet, dass** die Trenn- und Ablenkmittel ein Prisma, das dazu bestimmt ist, den Lichtstrahl in zwei sekundäre Lichtstrahlen zu trennen, und eine Kombination aus Spiegeln und/oder Linsen umfassen, die dazu bestimmt ist, den Pfad der sekundären Lichtstrahlen zu modifizieren.

8. Vorrichtung nach Anspruch 5, **dadurch gekennzeichnet, dass** die Anzeigemittel (12, 12', 12") mindestens ein Lichtquellenpaar, jeweils eine erste Lichtquelle, die den ersten Lichtstrahl emittiert, und eine zweite Lichtquelle, die den zweiten Lichtstrahl emittiert, umfassen.

9. Vorrichtung nach einem der Ansprüche 6 bis 8, **dadurch gekennzeichnet, dass** die Lichtquelle bzw. das Lichtquellenpaar ein Laser bzw. ein Laserpaar ist.

10. Vorrichtung nach einem der Ansprüche 1 bis 4, **dadurch gekennzeichnet, dass** die Anzeigemittel mindestens drei vertikal ausgerichtete Lichtsäulen (12, 12', 12") umfassen, jeweils eine erste Lichtsäule (12), die in Ausrichtung mit der ersten Innenfläche (13) und in Ausrichtung mit der zweiten Innenfläche (15, 15') angeordnet ist, eine zweite Lichtsäule (12'), die in Ausrichtung mit der zweiten Innenfläche (15, 15') und links oder rechts von der ersten Lichtsäule (12) angeordnet ist, und eine dritte Lichtsäule (12"), die in Ausrichtung mit der ersten Innenfläche (13) und näher an der Rückseite des Fahrzeugs als die erste Lichtsäule (12) angeordnet ist, wobei jede der Lichtsäulen (12, 12', 12") aus einer Vielzahl von Lichtpunkten gebildet wird, die in vertikaler Richtung ausgerichtet sind, wobei jeder der Lichtpunkte in einem aktivierten Zustand Licht emittieren kann und in einem deaktivierten Zustand kein Licht emittieren kann, und dadurch, dass die erste künstliche Horizontlinie (14) durch die virtuelle Gerade gebildet wird, die durch die höchsten oder niedrigsten aktivierten Lichtpunkte (17, 17") auf der ersten bzw. dritten Lichtsäule (12, 12") verläuft, und die zweite künstliche Horizontlinie (16) durch die virtuelle Gerade gebildet wird, die durch die höchsten oder niedrigsten aktivierten Lichtpunkte (17, 17") auf der ersten bzw. zweiten Lichtsäule (12, 12') verläuft.

11. Vorrichtung nach Anspruch 10, **dadurch gekennzeichnet, dass** jede der Lichtsäulen (12, 12', 12") eine lineare Anordnung von vertikal ausgerichteten lichtemittierenden Dioden umfasst, wobei jede der lichtemittierenden Dioden einen Lichtpunkt bildet.

12. Kraftfahrzeug (10), das mit einer Anti-Kinetose-Vorrichtung nach einem der vorhergehenden Ansprüche ausgestattet ist.

13. Kraftfahrzeug (10), das mit einer Anti-Kinetose-Vorrichtung nach einem der Ansprüche 5 bis 9 ausgestattet ist, **dadurch gekennzeichnet, dass** es mindestens eine Mittelsäule (11, 11') umfasst, wobei die Mittelsäule die Anzeigemittel (12, 12') trägt, wobei die Anzeigemittel dazu konfiguriert sind, den ersten Lichtstrahl auf eine hintere Seitenscheibe oder eine Verkleidung (13) der Hintertür des Fahrzeugs zu projizieren und den zweiten Lichtstrahl auf eine Innenfläche (15, 15') des Fahrgastraums, die im Wesentlichen senkrecht zu der Scheibe oder der Verkleidung (13) der Tür angeordnet ist, zum Beispiel die Rückenlehne eines der Vordersitze des Fahrzeugs, zu projizieren.

14. Verfahren zum Anzeigen von Lichtmarkierungen zum Bekämpfen der Kinetose, das die folgenden Schritte umfasst:
- Erfassen der Beschleunigungen eines Fahrzeugs entlang von 3 Achsen mittels eines dreiachsigen Beschleunigungsmessers und Senden entsprechender Signale an eine Steuereinheit;
- Bestimmen einer Beschleunigungsfrequenz entlang jeder der Achsen des Beschleunigungsmessers in Echtzeit und Vergleichen der bestimmten Beschleunigungsfrequenz und einer Schwellenfrequenz, unterhalb derer die Kinetose wahrscheinlich auftritt;
- in dem Fall, in dem die bestimmte Beschleunigungsfrequenz niedriger ist als die Schwellenfrequenz, Ansteuern durch die Steuereinheit von Anzeigemitteln (12, 12', 12") für Lichtmarkierungen, die dazu ausgelegt sind, mindestens eine erste künstliche Horizontlinie (14, 14') auf der Höhe einer ersten Innenfläche (13) des Kraftfahrzeugs und mindestens eine zweite künstliche Horizontlinie (16, 16') auf der Höhe einer zweiten Innenfläche (15, 15') des Kraftfahrzeugs zu bilden, wobei die erste und die zweite künstliche Horizontlinie (14, 16; 14', 16') senkrecht oder im Wesentlichen senkrecht zueinander sind, so dass die erste und die zweite künstliche Horizontlinie (14, 16; 14', 16') unabhängig von den Beschleunigungen des Fahrzeugs in einer horizontalen Ebene (A) senkrecht zum Gravitationsvektor ausgerichtet sind.

## Claims

1. An anti-motion sickness device fitted to a motor vehicle (10), **characterized in that** the anti-motion sickness device comprises:
- a triaxial accelerometer configured to detect the accelerations of the vehicle (10) along 3 axes and emit a corresponding acceleration signal;
- display means (12, 12', 12") of light markers capable of forming at least one first artificial horizon line (14, 14') at the level of a first internal surface (13) of the motor vehicle and at least one second artificial horizon line (16, 16') at a second internal surface (15, 15') of the motor vehicle, said first and second artificial horizon lines (14, 16; 14', 16') being perpendicular or substantially perpendicular to each other;
- a control unit capable of receiving the acceleration signals emitted by the accelerometer and of driving the display means (12, 12', 12") such that the first and second artificial horizon lines (14 , 16; 14', 16') are aligned in a horizontal plane (A), perpendicular or substantially perpendicular to the gravitational vector, whatever the accelerations of the vehicle, the control unit being moreover configured to process said acceleration signals, prior to controlling the display means (12, 12', 12"), so as to determine in real time an acceleration frequency along each of the axes of the accelerometer and to control said display means (12, 12', 12") only when said acceleration frequency is less than a threshold frequency below which the motion sickness is likely to occur.

2. The device according to claim 1, **characterized in that** the threshold frequency is equal to 25 Hz.

3. The device according to claim 1, **characterized in that** the threshold frequency is comprised between 4 and 8 Hz.

4. The device according to claim 1, **characterized in that** the threshold frequency is equal to 0.5 Hz for the accelerations along a longitudinal axis, corresponding to the front-rear direction of the vehicle, and along a lateral axis, corresponding to the right-left direction of the vehicle, and is equal to 2 Hz for the accelerations along a vertical axis, corresponding to the up-down direction of the vehicle.

5. The device according to any of the preceding claims, **characterized in that** the display means (12, 12', 12") are capable of emitting at least two light beams of rectilinear shape, respectively a first light beam projected onto the first internal surface (13) and forming the first artificial horizon line (14, 14'), and a second light beam projected onto the second internal surface (15, 15') and forming the second artificial horizon line (16, 16').

6. The device according to claim 5, **characterized in that** the display means (12, 12', 12") comprise at least one light source emitting a main light beam, and means for separating and deflecting said main light beam into two secondary light beams.

7. The device according to claim 6, **characterized in that** the separation and deflection means comprise a prism intended to separate the light beam into two secondary light beams and a combination of mirrors and/or lenses intended to modify the trajectory of said secondary light beams.

8. The device according to claim 5, **characterized in that** the display means (12, 12', 12") comprise at least one pair of light sources, respectively a first light source emitting the first light beam and a second light source emitting the second light beam.

9. The device according to any of claims 6 to 8, **characterized in that** the light source, respectively the pair of light sources, is a laser, respectively a pair of lasers.

10. The device according to any of claims 1 to 4, **characterized in that** the display means comprise at least three light columns (12, 12', 12") vertically oriented, respectively a first light column (12) arranged in alignment with the first internal surface (13) and in alignment with the second internal surface (15, 15'), a second light column (12') disposed in alignment with the second internal surface (15, 15') and to the left or right of the first light column (12), and a third light column (12") arranged in alignment with the first internal surface (13) and closer to the rear of the vehicle than the first light column (12), each of the light columns (12, 12', 12") being formed of a plurality of light points aligned in the vertical direction, each of the light points being able to emit light in an activated state and not to emit light in a deactivated state, and **in that** the first artificial horizon line (14) is formed by the virtual line passing through the highest, or lowest, activated light points (17, 17") respectively on the first and third light columns (12, 12") and the second artificial horizon line (16) is formed by the virtual line passing through the highest or lowest activated light points (17, 17'), respectively on the first and second light columns (12, 12').

11. The device according to claim 10, **characterized in that** each of the light columns (12, 12', 12") comprises a linear array of light-emitting diodes aligned vertically, each of the light-emitting diodes forming a light point.

12. A motor vehicle (10) equipped with an anti-motion sickness device according to any of the preceding claims.

13. The motor vehicle (10) equipped with an anti-motion sickness device according to any of claims 5 to 9, **characterized in that** it comprises at least one central pillar (11, 11'), said central pillar supporting the display means (12, 12'), said display means being configured to project the first light beam onto a rear side window or a rear door panel (13) of the vehicle and to project the second light beam onto an internal surface (15, 15') of the passenger compartment arranged substantially perpendicular to said window or said door panel (13), for example the back of one of the front seats of the vehicle.

14. A method for displaying light markers intended to combat motion sickness, comprising the following steps of:
- detecting using a triaxial accelerometer of the accelerations of a vehicle along 3 axes and sending corresponding signals to a control unit;
- determining in real time of an acceleration frequency along each of the axes of the accelerometer and comparing between the determined acceleration frequency and a threshold frequency below which motion sickness is likely to occur;
- in the case where the determined acceleration frequency is lower than the threshold frequency, driving by the control unit of display means (12, 12', 12") light markers capable of forming at least one first artificial horizon line (14, 14') at a first internal surface (13) of the motor vehicle and at least one second artificial horizon line (16, 16') at a second internal surface (15, 15') of the motor vehicle, said first and second artificial horizon lines (14, 16; 14', 16') being perpendicular or substantially perpendicular to each other, such that the first and second artificial horizon lines (14, 16; 14', 16') are aligned in a horizontal plane (A), perpendicular to the gravitational vector, whatever the accelerations of the vehicle.
